# EUROPEAN PATENT APPLICATION

(11) **EP 2 829 225 A1**
(43) Date of publication of application: **28.01.2015**
(21) Application number: 12871914.3
(22) Date of filing: 20.03.2012
(51) Int. Cl.: A61B 5/01, A61B 5/145, A61B 5/1477

(54) **METHOD AND DEVICE FOR NON-INVASIVE CHECKING OF THE GLUCOSE LEVEL IN THE BLOOD**

(71) Applicant: BIRMELE, Kalyna Maya, Rocky River OH 44116 (US)
(72) Inventor: KHOKHOEV, Elbrus Marklenovich, Vladikavkaz 362007 RSO-Alania (RU); KHOKHOEV, Timur Elbrusovich, Vladikavkaz 362000 RSO-Alania (RU); TZALLAEV, Denis Batrazovich, Vladikavkaz 362044 RSO-Alania (RU)
(74) Representative: Von Kreisler Selting Werner - Partnerschaft von Patentanwälten und Rechtsanwälten mbB
(86) International application number: PCT/RU2012/000194
(87) International publication number: WO 2013/141734

(57) **Abstract**

**SUMMARY**

This invention medical technology relates to medical devices more specifically to method of measuring and monitoring glucose levels in the blood.

Throughout the described process of this invention of non-invasive monitoring of blood glucose levels, providing temperature measurement and analysis of various organs of the body, characterized in that the temperature measurement is performed equally in insulin dependent and non-insulin dependent tissues of the body; while the data of the blood glucose level is obtained by analyzing the relationship between glucose level and the temperature difference between insulin dependent and non-insulin dependence in the bodies organs.

This newly invented device implementing this method includes a measuring unit, a unit for processing the data received, and a unit for inputting/outputting instructions, and displaying the results of data-processing. It is characterized in that the temperature-measuring unit is in the form of a thermo imager, and the unit for processing the data produced can analyze the temperature difference recorded in the insulin dependent and non-insulin independent tissues of the body, and comparing the value obtained from the correlation tables reflecting the physiological characteristics of different categories of the population.

## Description

The invention relates to medical devices, and specifically to methods for monitoring glucose and can be used for non-invasive determination of blood glucose levels.

It is known that the control of blood glucose levels is an indispensable procedure in medical diagnosis, in particular it is important for people with diabetes. Controlling blood glucose levels is an indispensable procedure in medical diagnosis and treatment, and is especially true for people with diabetes. The approximate number of diabetic patients in the following countries is most alarming and is expected to drastically increase:

### CURRENT DIAGNOSTIC METHODS

Currently, the most common method of determining the level of glucose in the blood is taking a blood sample and applying this blood soaked enzyme colorimetric strip or an electrochemical probe. Usually this procedure involves pricking the finger. For persons suffering from diabetes and need internally measured blood glucose level several times a day, the use of this method brings significant discomfort and increased risk of infection.

Based on presently known solutions, based on non-invasive methods, still none of the known methods provide sufficiently high accuracy of measurements of glucose. Furthermore, all known methods require the use of very complex machinery.

The non-invasive known methods in use currently do not provide adequate high accuracy measurements of glucose levels, and require the use of extremely complex equipment. A brief description of six U.S. patented methods includes:
a. For instance U.S. Patent No. 5119819. This non-invasive method of monitoring blood glucose is based on measuring the speed of sound in blood, and analyzing the difference between the primary and the reflected ultrasonic pulse. The blood temperature is also measured, because the velocity of sound propagation in the blood depends on the blood temperature. The devise in this method is a miniature sound generator and a reflecting mirror placed on the outer and inner sides of the earlobe; with a monitor to detect the temperature change of the blood, and the rate of passage of the sound waves to determine the concentration of glucose in the blood. A serious drawback in this method is that it is capable of displaying only the dynamics of fluctuations in the level of glucose (increase/decrease), but unable to determine the absolute values of glucose.
b. U.S. Patent No. 5771891. This non-invasive method of measuring glucose in blood requires an electrical stimulation of the endogenous tissue which is then recorded, and the resulting response to the stimulation analyzed. It is assumed that this method can control the contents of the various components in the blood, including glucose. Apart from the fact that holding such an analysis requires considerable computing resources, the results of measurements in a nonlinear manner depend on a large number to external factors; such as body temperature, pressure, and other mental conditions that distort the results of the measurements.
c. U.S. Patent No. 5795305 and Patent No. 5924996. To determine the concentration of glucose in the blood this method requires a highly accurate temperature measurement of a portion of the body-infrared radiation and the heat conduction area of the skin at the determined site. This analysis is primarily based on mathematical extrapolation methods, and does not take into account the impact of external factors on the change in body temperature.
d. U.S. Patent No. 5941821 and Patent No. 6049728. These two patents propose that blood glucose levels are determined based on an analysis of the effects of the acoustic pulse, in particular the skin and the subcutaneous tissue of the patient. This method provides non stable results for it does not take into account individual differences in the skin of the patient.

It should be stated that a technical solution to Russian Patent, No. 2376927, which proposes to measure directly three to five options to reduce the chance of error for each of the above stated methods, do not individually always work.

Determining the glucose level based on the analysis of the temperature difference between arterial and venous blood is a known method (RF Patent No. 2180514). This process involves a continuous monitoring of glucose concentration in the blood by measuring the area of the surface veins in the head part of the body temperature, and conducted via heat flow sensors of the measuring device, and the glucose concentration (Xg *) is determined by the formula Xg * = X1 * + X2 * where X₁*= W_{TΠ}(s)X_{T}*, X₂*= K_{Π}W_{TΠ}(s)X_{Π}*, Γ 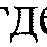 e X_{T}*, where X_{T}*- dimensionless temperature deviation from the steady-state value, X_{Π}* - dimensionless deviation from the steady heat flow values W_{TΠ}(s) = 1/(T_{TΠ}s+1) - transfer function of the concentration of glucose in the blood temperature and heat flow, T_{TΠ} - experimentally determined time constant transient K_{Π} - experimentally determined dimensionless coefficient, s = d/dt - the differentiation operator.

As can be seen from the claims, the method is designed for long term monitoring of the accumulation of experimental data, and can be used in clinical medicine; but it is extremely difficult to use at home. The method is chosen as a prototype of the claimed invention.

Thus, numerous methods are known to determine the blood glucose level by calculating the correlation between some of the indirect parameters: measuring the speed of sound, conductivity, spectral analysis, the acetone content in exhaled air, perspiration and measuring the composition, etc. All of these techniques use extremely complex methods depending on correlation, which are executed only under specific conditions and have low accuracy.

### THE NON-INVASIVE TECHNOLOGY

This new invention uses a simple, but reliable method, and a non-invasive apparatus (instrument) to accurately determine the blood glucose levels. The technical result is achieved by the use of simple, almost linear relation between glucose levels and temperature differences in insulin-dependent and non-insulin dependent organs of the body. This procedure uses a device consisting of a temperature measuring unit, the received data processing unit, and the I/O commands and display results of data processing.

The concept and accuracy of this invention is based on the fact that certain organs of the human body metabolize glucose, without the help of insulin, i.e. they are insulin independent. These include brain cells, lens, retina, nerve endings. To sustain other tissues and organs with glucose requires insulin.

A shortage of insulin in the body increases the level of glucose in the blood, which leads to increased insulin-independent work of the body's tissues, followed by the release of heat and increase in temperature. Thus insulin-dependent tissues do not receive sufficient glucose, and their biological activity is reflected in lower temperatures.

The claimed method of non-invasive monitoring of blood glucose levels is based on the determination of correlation between the temperature difference between insulin dependent and non-insulin dependent bodies; while this dependence is almost always unique and can characterize the level of glucose in the blood.

In other words, the present method is sufficient to perform the measurement and analyses of a single parameter, since this parameter in the body always has a unique dependence, except where specifically indicated in some rare cases where the method cannot be applied (local inflammation, serious injury, or lack of enforcement, etc.).

The recently invented procedure uses a non-invasive blood glucose meter designed for temperature control and calculation of blood glucose level on the basis of insulin dependent radiation temperature difference (oral mucosa, skin eyeball, etc.) and non-insulin dependent organs (fundus lens, retina adjacent to retinal tissue of the vitreous body).

The distinctive feature of this device is that it is configured to measure the biological activity of the tissues, which is a consequence of the assimilation of glucose by the cell.

Meanwhile, in the presently known technical solutions the main role in insulin therapy is accomplished by correcting the level of glucose in the blood, and as a consequence to ensure the proper nutrition of the cells by transporting glucose and not the measurement of direct sustenance of the cells. In this manner, in the final analysis the technique used today is correct to support normal glycemia in the blood, and indirectly related to the analysis of biological activity of insulin dependant tissues.

Currently, the amount of glucose in the blood in relation to the introduction of the organism-"bread units" is interpreted as lack of, or overabundance of insulin. According to this, they support the biological activity of insulin dependent organs in a state of normal glycemia.

When using an indirect assessment of the biological activity it is difficult to assess the impact of glycemia on other hormones in the endocrine system, including contra insular such as adrenaline, cortisol, amelin, glucagon; and other that inhibit the action of its own, as well as injected insulin.

Using this new invention, based on the analysis of the biological activity of the tissues, it eliminates similar problems, since it directly measures the state of insulin dependent and insulin non-dependent tissues (final consumers glucose), and based on correlation of dependence it displays values of normal glycemia of the organism.

### DETAILED DESCRIPTION INVOLVING GRAPHIC MATERIAL

Incorporating graphic material in the following detailed description presents a clearer understanding of the new procedure, and the non-invasive blood glucose meter. There are three main steps in this new invention.

### Section 1. Temperature Measurement and Assessment

Block 100: Temperature measurement of two bodies: the insulin dependent and insulin non-dependent.

Block 101: Assessment of the two values obtained.

Block 103: Comparison of estimates obtained from the temperature dependence of reference data of the organisms obtained when taken from a particular category of persons with normal glycemia.

Block 104: Displays the result of comparison in computer readable format, graphical format, audio signal format, or in any other format. The reference data, summarized in a chart or table, is selected from the category of persons closest to that category which refers to a particular patient. These categories are based on physiological differences between patients and take into account mainly, race, gender, age and other exogenous and endogenous factors affecting the metabolic processes in the human body.

### Section 2. Chart explaining the use of the non-invasive Blood Glucose Meter

Block 200: The block temperature measurement with high resolution (in the preferred embodiment, apparatus used with a resolution of 0.01 °C and above).

Block 201 and 202: Temperature sensors as one of the choices presently used is an imager device.

Block 203: A processing unit analyzing data obtained from Block 200, and comparing it with reference data.

Block 204: Commanding unit (control unit) configured with ability to command input into Block 203, and output the results of analysis on the display device 205 (display, speaker, printer, etc.).

### Section 3. Flow chart for obtaining measurement results of blood glucose level

At step 301 the values obtained of temperature t1 (non-insulin body temperature) and t2 (insulin body temperature). Next, transmitting data of temperatures to Step 302, where they are compared with reference values, or in Step 303, wherein the values are calculated by indicated further functions. At Step 304 convert the values obtained in the measure of glucose levels.

Constructive implementation of the device, the non-invasive blood glucose meter, depends on the desired accuracy of its readings. In particular, Block 200 may be represented by a matrix, or a dotted imager with a transparent window placed on the pupil and the vitreous body of the eyeball, with the use of cooled or non-cooled matrices able to determine the temperature of the fundus, or vitreous of the eyeball with a resolution of at least 0.01 °C . Block 203 is a hardware computing module configured to support the software that controls the temperature sensor (thermal imager), analyzing the thermo gram of the eyeball and calculating the dependence of temperature of the glucose using correlation tables or graphs on key points. Block 204 is designed as a control device that provides input commands from a keyboard or other input device, and outputting the results to the display device. Blocks may be performed as part of a single device or as a set of separate devices. Exchange of data between the blocks can be performed by wired transmission or wireless manner (Bluetooth, WiFi, etc.).

Block 200: The instrument is brought close to the eye of the patient. If an imager is not being used, it is to be supplemented by infrared temperature sensors as shown in Block 201 and 202. The device is ready to be used.

The preferred method of usage of the invented instrument was used with a thermal imager; which simultaneously recorded the temperature in the key areas of the lens, the vascular connection of the eyeball, the fundus, and the temperature of the skin located in the area adjacent to the eye. In the alternate method of examination was conducted with the help of a temperature imager at two points or cloud point on insulin, and non-insulin dependant organs.

Processing the data obtained by measurement of temperature is performed in accordance with an algorithm through which is established the level of glucose in the blood by comparing the received data which was stored in Block 203, based on corrective coefficients established for a specific person, or the calculation values of glucose in the blood with the use of the function G₁=fₙ(t₁,t₂)*(k₁...kₙ) wherein G₁ level of glucose, t₁= value of temperature in non-dependent insulin organ, t₂= value of temperature in insulin dependent organ. k₁...kₙ = correction coefficients for the various categories of the population.

This newly invented procedure using this unique instrument to monitor blood glucose may be used in the home as well as clinically, with highly accurate diagnosis, risk free of any infection, and totally pain free for it does not require pricking the skin.

**Procedural Formula Using Non-invasive Device**

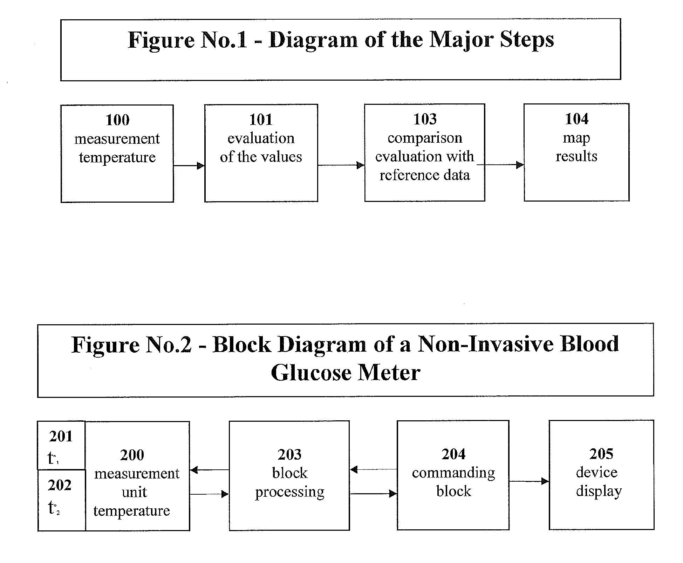

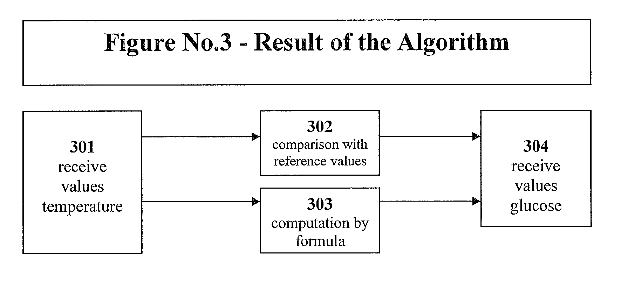

## Claims

1. Non-invasive method for monitoring blood glucose levels, providing temperature measurement and analysis of various organs in the body, **characterized in that** the temperature measurement is carried out in respect of insulin-dependent and insulin-independent tissues of the body, wherein data on the blood glucose level is obtained by analyzing the relationship between glucose level and insulin dependent temperature difference in body organs and non-insulin.

2. According to claim 1, this **characterized in that** the analysis depends on the use of correlation tables containing reference data on the level of glucose in populations that differ in physiological characteristics based on race, gender, age and profession of the patient.

3. According to claim 1, this method wherein the dependency is established by the use of algorithms in which, based on the initial schedule measurements, the functions are calculated on the formula G₁=fₙ(t₂,t₁)*(k₁...kₙ) wherein G₁ = value of glucose, t1= value of the temperature in insulin non-dependent organ, t2 = value of temperature in insulin dependent organ, k1 ... kn = correction coefficients for the various categories of the population.

4. Device for non-invasive glucose monitoring of the blood, including a measuring unit, a block of received data processing unit, and a block of input/output commands and display results of data processing, **characterized in that** the temperature measuring unit is configured as a thermal imager, but
the block receiving data processing unit configured to analyze the difference of temperatures recorded in insulin dependent and non-insulin dependent tissues of the body, and comparing the value obtained with the correlation tables reflecting the physiological characteristics of the different categories of the population.
